# EUROPEAN PATENT SPECIFICATION

(11) **EP 3 938 027 B1**
(45) Date of publication and mention of the grant of the patent: **20.09.2023**
(21) Application number: 20718042.3
(22) Date of filing: 13.03.2020
(51) Int. Cl.: A61N 1/05, A61N 1/20

(54) **SYSTEM FOR ACCELERATED RECOVERY FROM DIRECT CURRENT (DC) NERVE BLOCK USING REPOLARIZATION**
SYSTEM ZUR BESCHLEUNIGTEN WIEDERHERSTELLUNG VON GLEICHSTROMNERVENBLOCKADEN MITTELS REPOLARISATION
SYSTÈME DE RÉCUPÉRATION ACCÉLÉRÉE D'UN BLOC NERVEUX EN COURANT CONTINU (DC) PAR REPOLARISATION

(30) Priority: 15.03.2019 US 201962818773 P
(43) Date of publication of application: 19.01.2022
(73) Proprietor: CASE WESTERN RESERVE UNIVERSITY, Cleveland, Ohio 44106 (US)
(72) Inventor: VRABEC, Tina L., Cleveland, Ohio 44106 (US); KILGORE, Kevin L., Cleveland, Ohio 44106 (US); WAINRIGHT, Jesse S., Cleveland, Ohio 44106 (US); BHADRA, Niloy, Cleveland, Ohio 44106 (US)
(74) Representative: Marks & Clerk LLP
(86) International application number: PCT/US2020/022583
(87) International publication number: WO 2020/190694

(56) References cited:
- WO-A1-2013/188753
- WO-A1-2017/062272
- WO-A1-2018/075473
- US-A1- 2016 101 286
- US-A1- 2017 050 024
- US-A1- 2018 280 691

## Description

### Technical Field

The present disclosure relates generally to electrical block of transmission of neural signals and, more specifically, to accelerated recovery from direct current (DC) nerve block and/or enhancing condition using repolarization.

### Background

Direct current (DC) can be applied to a nerve to generate a complete nerve block. Using conventional electrodes (e.g., platinum electrodes), however, applying DC to a nerve can lead to the generation of irreversible reaction products that can damage the nerve. New electrodes, such as the separated interface nerve electrode (SINE), have been developed that are designed to reduce or eliminate the damaging effects of the irreversible reaction products when the DC is applied to the nerve. For example, the SINE can separate the electrode from the nerve using a biocompatible, ionically conducting medium, which isolates the damaging reactions in a vessel away from the nerve. However, when the DC is applied to the nerve to achieve complete block for a prolonged period of time using the new electrodes, a delay is seen in the reversibility of the block and recovery of the neural response, requiring a substantial recovery period. In many cases, it would be advantageous for the recovery period to be reduced so that neural response to be restored more quickly. Examples of prior-art documents disclosing similar systems and/or method can be found, for instance in US2018/280691 A1, WO2018/075473 A1, WO2017/062272 A1, US2017/050024 A1, US2016/101286 A1, and WO2013/188753 A1.

### Summary

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

### Brief Description of the Drawings

The foregoing and other features of the present disclosure will become apparent to those skilled in the art to which the present disclosure relates upon reading the following description with reference to the accompanying drawings, in which:
FIG. 1 is a schematic diagram showing an example of a system that can be used to achieve accelerated recovery from direct current (DC) nerve block using repolarization in accordance with an aspect of the present disclosure;
FIG. 2 shows an example of the system of claim 1 with the waveform generator receiving input from a controller;
FIG. 3 is a process flow diagram illustrating a method for accelerating recovery from DC nerve block using repolarization in accordance with another aspect of the present disclosure;
FIG. 4 is a process flow diagram illustrating a method for enhancing a response of the nerve without blocking conduction in the nerve in accordance with yet another aspect of the present disclosure;
FIG. 5 shows an experimental set-up using a separated interface nerve electrode (SINE);
FIG. 6 shows examples of cathodic (top) and anodic (bottom) block, illustrating that both cathodic and anodic currents can cause block, but block thresholds are lower for cathodic;
FIG. 7 shows an example of recovery from complete cathodic block without recovery acceleration; and
FIG. 8 shows examples of recovery from complete cathodic block with recovery acceleration.

### Detailed Description

### I. Overview

Direct current (DC) can be applied to a nerve to generate a complete nerve block. A problem with applying DC to the nerve with a traditional electrode is the generation of reaction products, which can damage the nerve. Newer electrodes, such as the separated interface nerve electrode (SINE), are designed to reduce or eliminate the damaging effects of the irreversible reaction products when the DC is applied to the nerve. When DC is applied for a prolonged period of time with these newer electrodes, such as the SINE, however, a delay is seen in the reversibility of the block and recovery of the neural response, requiring a substantial recovery period. The recovery period can be reduced by applying a repolarizing waveform (e.g., a subthreshold DC of the opposite polarity) after application of the DC at or above the block threshold.

Accordingly, the present disclosure is related to the accelerated recovery from direct current (DC) nerve block using repolarization. Specifically, the systems and methods described herein can be used to accelerate the recovery of nerve conduction post-block by applying a sub-threshold reversed-polarity DC waveform to the nerve. Additionally, changing the electric field caused by the sub-threshold reversed-polarity DC waveform enhances nerve conduction, even when applied alone and/or when the nerve is still blocked. Using the systems and methods of the present disclosure, nerve block can be administered to a patient, completely numbing some portion of the patient's body when needed, for example, and the numbing can be reversed instantaneously (or at least nearly immediately with a reduced response time).

### II. Systems

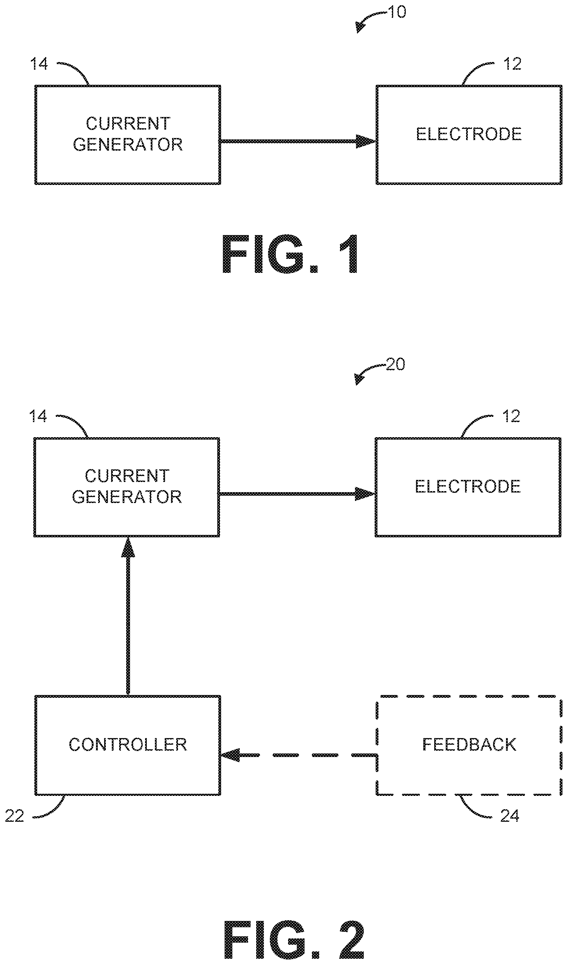

An aspect of the present disclosure can include a system 10 (FIG. 1) that can be used to achieve accelerated recovery from direct current (DC) nerve block using repolarization. DC can be applied to a nerve to generate a complete nerve block (e.g., with an anodic or cathodic amplitude at or above the threshold for block). Newer electrodes, like the separated interface nerve electrode (SINE), have been designed to reduce or eliminate the damaging consequences of irreversible reaction products. Accordingly, the newer electrodes allow DC block to be administered for a prolonged period of time, but when the DC is applied for a prolonged period of time, however, a delay is seen in the reversibility of the block and recovery of the neural response, requiring a substantial recovery period. The system 10 can reduce the recovery period by applying a repolarizing waveform (e.g., a subthreshold DC of the opposite polarity - anodic if the block is cathodic, cathodic if the block is anodic). As a practical example, using the system 10, nerve block can be administered to a patient, completely numbing some portion of the patient's body when needed, for example, and the numbing can be reversed instantaneously (or at least nearly immediately with a reduced response time). Additionally, the reverse polarity waveform applied by the system 10 can enhance neural activity, even when applied alone and/or when the nerve is still blocked.

The system 10 includes at least one or more electrodes (shown as electrode 12, but it will be understood that the electrodes are not restricted to a single device) and one or more waveform generators (shown as current generator 14, but it will be understood that the waveform generator is not restricted to only a single device that only generates current). The one or more electrodes (including electrode 12) can be coupled to the current generator 14 for signal transmission therebetween. The coupling can be a wireless coupling, a wired coupling, or a combination of wired coupling and wireless coupling. As an example, the coupling can be a wired coupling so that a signal can be transmitted between the current generator 14 and the electrode 12.

The current generator 14 can be configured or programmed to generate the electrical signal, for example a DC, configured with an amplitude sufficient to cause block in at least a portion of neural tissue (e.g., sensory neurons, motor neurons, autonomic neurons, enteric neurons, interneurons, central nervous system neurons, etc.). Accordingly, the current generator 14 can be any device configured or programmed to generate the specified electrical signal. One example of a current generator 14 is a battery-powered, portable generator. Another example of a current generator 14 is an implantable generator (IPG). It will be appreciated that the current generator 14 can include additional components to selectively configure the current waveform, such as an amplitude modulator (not shown). As an example, the current generator 14 can be configured or programmed to generate a DC waveform having monophasic waveform or a biphasic waveform, with one phase cathodic and one anodic. As another example, the current generator 14 can be configured or programmed to generate a charge balance polarizing current (CBPC) waveform.

In some instances, the generated DC waveform can have an anodic polarity or a cathodic polarity, and an amplitude sufficient to cause the DC block. For block, the DC can have an amplitude that is at least a block threshold. The block threshold for different types of nerves of different sizes may be different. The DC used for block can be cathodic (positively charged) with a cathodic amplitude of at least the cathodic block threshold or anodic (negatively charged) with an anodic amplitude of at least the anodic block threshold. The DC block can be provided as hyperpolarization block or depolarization block. Hyperpolarization block is caused by the accumulation of negative charges and/or loss of positive charges within a cell, lowering its membrane potential below its resting potential, caused by an anodic current. Depolarizing block is caused by the accumulation of certain positively charged ions within a cell, caused by a cathodic current. The DC used for block can be administered for a time. As a non-limiting example, the time can be more than 2 minutes. As another non-limiting example, the time can be more than 5 minutes. As yet another non-limiting example, the time can be more than 10 minutes.

The electrode 12 can be configured to deliver the electrical signal to neural tissue. For example, the electrode 12 can be coupled to the current generator 14 to receive the electrical signal and deliver the electrical signal to the neural tissue to provide the block for the time. The electrode 12 can be a newer electrode design, which can be designed to avoid irreversible Faradaic reactions, like hydrogen evolution, oxygen evolution, chlorine evolution, or the like, at the high DC charge required to deliver the nerve block. For example, the electrode 12 can use a separated interface, separating the electrode and the nerve. As another example, the electrode 12 can utilize high capacitance electrode materials and/or high charge capacity electrode materials. Specific examples of electrodes that can be used as the electrode 12 include a separated interface nerve electrode (SINE), a carbon coated platinum electrode, a woven cloth carbon electrode, a carbon slurry electrode, or the like (e.g., designed as described in at least one of U.S. 9,008,800, U.S. 9,496,621, WO 2019/133783, WO 2019/133784, U.S. 9,387,322, or U.S. 10,195,434).

After the time, the current generator 14 can be configured or programmed to generate another electrical signal with another amplitude (different from the amplitude of the original electrical signal) for another time. The other electrical signal can be a DC or a CBDC, configured with the other amplitude that is sub-threshold (i.e., does not cause block in at least a portion of neural tissue) and of the opposite polarity (also referred to as reversed polarity) from that of the originally generated DC waveform. In other words, the other amplitude can be of the reversed polarity than the amplitude of the originally generated DC waveform. For example, if the original DC waveform has an anodic polarity, the other electrical signal can have a cathodic polarity. Similarly, if the original DC waveform has a cathodic polarity, the other electrical signal can have an anodic polarity.

The sub-threshold amplitude can be any value less than the threshold (e.g., either the cathodic threshold or the anodic threshold, depending on the polarity of the other electrical signal). For example, the sub-threshold amplitude can be from 0.1 % of the threshold to 99.9 % of the threshold. As another example, the sub-threshold amplitude can be a value less than 85 % of the threshold. As further example, the sub-threshold amplitude can be a value less than 50 % of the threshold. As yet another example, the sub-threshold amplitude can be a value less than 30 % of the threshold. It should be noted that the value of the sub-threshold amplitude may be based on the type of nerve and/or the location of the nerve. For example, a smaller nerve may require a different amplitude value than a larger nerve.

The electrode 12 can receive the other electrical signal from the current generator 14 and deliver the other electrical signal to the neural tissue for the other time. The other signal can cause the one or more nerves within the neural tissue to enter a repolarization cycle to accelerate the recovery from the block (in other words, accelerate recovery time for the nerve). In some instances, neural activity can be fully restored in the nerve after the second time.

The recovery time can be markedly reduced when the sub-threshold, reversed polarity DC is applied. It should be understood that the recovery time can be reduced to be less than it would be with application of the DC signal without the other DC signal. In some instances, the recovery time can be from 10 minutes to about 0 seconds (nearly instantaneous). For example, the recovery time for the nerve can be less than 1000 s. As another example, the recovery time for the nerve can be less than 500 s. As a further example, the recovery time for the nerve can be less than 250 s. The recovery time may be based on the type of nerve and/or the location of the nerve. For example, a smaller nerve may require a different recovery time than a larger nerve.

The reversed polarity, sub-threshold other electrical signal can preserve the reversibility of DC nerve block when the DC nerve block is applied for a prolonged period of time. Using the reversed polarity, sub-threshold other electrical signal can be used, for example, when a DC nerve block is delivered to provide complete numbing of a portion of a patient's body, using the reversed polarity, sub-threshold other electrical signal can ensure that it is always known that the numbing can be reversed whenever it is desired.

Additionally, applying an electrical signal, either for block or repolarization, can change the electrical field in proximity to the nerve. When the blocking electrical signal is applied, the electric field can change to block the neural activity (e.g., conduction). When the sub-threshold electrical signal is applied, the electric field can change to one that provides a positive effect on nerve conduction. In some instances, application of the sub-threshold electrical signal can have an enhancement effect on neural activity, including nerve conduction. The sub-threshold electrical signal, in some instances, can have an enhancement effect on neural activity when applied by itself without blocking. The sub-threshold electrical signal, in other instances, can have an enhancement effect on neural activity when applied in conduction with a blocking electrical signal. The sub-threshold electrical signal, in still other instances, can have an enhancement effect on neural activity when applied after application of a blocking signal.

As shown in FIG. 2, the current generator 14 can be coupled to one or more controllers (shown as a single controller 22 in FIG. 2). The controller(s) 22 can be implantable and/or external. The controller(s) 22 can be configured to set one or more parameters of the electrical signal and/or the other electrical signal delivered by the current generator 14. The parameters can include polarity, amplitude, timing, and the like, for the electrical signal and/or the other electrical signal. It should be noted that one or more of the parameters will be different for each of the electrical signal and the other electrical signal.

In some instances, the controller(s) 22 can be programmed with set values for the one or more parameters of the electrical signal and/or the other electrical signal. In other instances, the controller(s) 22 can alter a value of the one or more parameters based on feedback 24. For example, the feedback 24 can be a user input (in this example, the controller 22 can have safety concerns programmed therein to prevent the one or more parameters to be adjusted to an unsafe or ineffective level). As another example, the feedback 24 can be from a sensor within the subject's body either before delivery of the electrical signal and the other electrical signal or after delivery of the electrical signal.

For example, the controller(s) 22 can be configured to program the current generator to generate the electrical signal with an amplitude and a polarity for a first time. The amplitude can be at least a threshold value (to provide block) and the polarity can be either anodic or cathodic. These parameters can be pre-programmed into the controller 22 or set according to a user input. The controller 22 can also be configured to program the current generator to generate the electrical signal with another amplitude and a reverse polarity for a second time. The other amplitude can be less than a threshold value (so not to provide block) and the polarity can be the reverse of that of the electrical signal - e.g., if the polarity of the electrical signal is either anodic or cathodic, the polarity of the other electrical signal is either cathodic or anodic. These parameters can be pre-programmed into the controller 22 or set according to a user input. For example, the amplitudes, polarities, and/or time of application of the electrical signal and/or the other electrical signal can be preprogrammed into the controller, but the start time of application of the other electrical signal can be set according to a user input.

### III. Methods

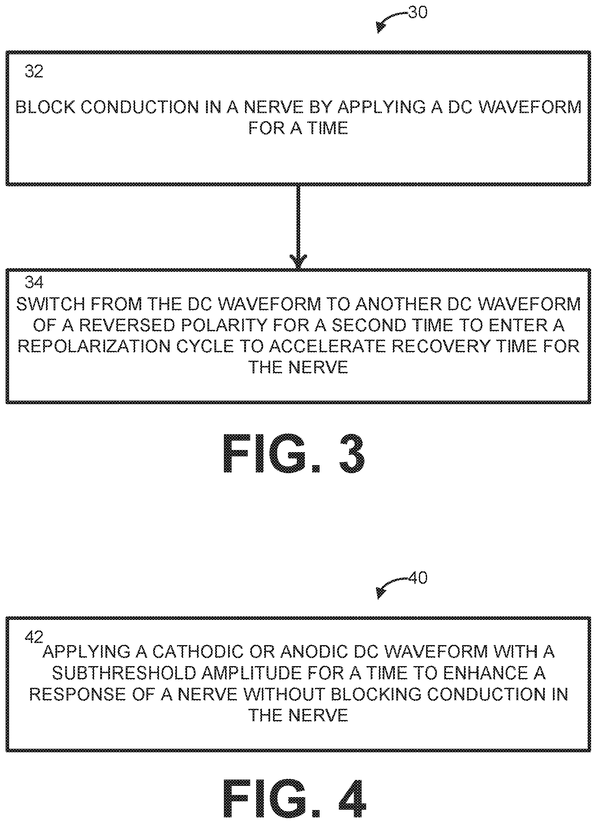

Another aspect of the present disclosure, not being part of the invention, can include methods 30 and 40 for achieving the effects of applying a repolarization DC current to a nerve, as shown in FIGS. 3 and 4. The methods 30 and 40 can be executed using the systems 10 or 20 shown in FIGS. 1 and 2, using the electrode(s) 12 designed to avoid Faradaic reactions, like hydrogen evolution, oxygen evolution, chlorine evolution, or the like, when delivering a large charge to neural tissue. For example, the electrode(s) 12 can utilize a saline interface. As another example, the electrode(s) 12 can utilize high capacitance electrode materials. Specific examples of electrodes that can be used as the electrode(s) 12 include a separated interface nerve electrode (SINE), a carbon coated platinum electrode, a woven cloth carbon electrode, a carbon slurry electrode, or the like.

For purposes of simplicity, the methods 30 and 40 are shown and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the methods 30 and 40.

Referring now to FIG. 3, illustrated is an example of a method 30 for accelerating recovery from DC nerve block using repolarization. The reversed polarity, sub-threshold other electrical signal can preserve the reversibility of DC nerve block when the DC nerve block is applied for a prolonged period of time. At Step 32, conduction in a nerve (or general neural activity) can be blocked by applying a DC waveform for a time (e.g., a DC or a CBDC generated by current generator 14 and applied by electrode 12). The DC waveform can have an amplitude of at least a block threshold. At Step 34, the DC waveform can be switched (e.g., according to controller 22) to another DC waveform of a reversed polarity for a second time (e.g., a DC or a CBDC generated by current generator 14 and applied by electrode 12) to enter a repolarization cycle to accelerate recovery time for the nerve. The other DC waveform can be a sub-threshold DC waveform of the reversed polarity that does not cause block.

The sub-threshold amplitude can be any value less than the threshold (e.g., either the cathodic threshold or the anodic threshold, depending on the polarity of the other electrical signal). For example, the sub-threshold amplitude can be from 0.1 % of the threshold to 99.9 % of the threshold. As another example, the sub-threshold amplitude can be a value less than 85 % of the threshold. As further example, the sub-threshold amplitude can be a value less than 50 % of the threshold. As yet another example, the sub-threshold amplitude can be a value less than 30 % of the threshold. It should be noted that the value of the sub-threshold amplitude may be based on the type of nerve and/or the location of the nerve. For example, a smaller nerve may require a different amplitude value than a larger nerve.

The recovery time can be markedly reduced when the sub-threshold, reversed polarity DC is applied. It should be understood that the recovery time can be reduced to be less than it would be with application of the DC signal without the other DC signal. In some instances, the recovery time can be from 10 minutes to about 0 seconds (nearly instantaneous). For example, the recovery time for the nerve can be less than 1000 s. As another example, the recovery time for the nerve can be less than 500 s. As a further example, the recovery time for the nerve can be less than 250 s. The recovery time may be based on the type of nerve and/or the location of the nerve. For example, a smaller nerve may require a different recovery time than a larger nerve.

Referring now to FIG. 4, illustrated is another example of a method 40 for enhancing a response of the nerve using repolarization without blocking conduction in the nerve. At Step 42, a cathodic or anodic DC waveform with a subthreshold amplitude can be applied for a time to enhance a response of a nerve without blocking conduction in the nerve. The response of the nerve can be, for example, conduction.

Applying an electrical signal, either for block or repolarization, can change the electrical field in proximity to the nerve. When the blocking electrical signal is applied, the electric field can change to block the neural activity (e.g., conduction). When the sub-threshold electrical signal is applied, the electric field can change to one that provides a positive effect on nerve conduction. In some instances, application of the sub-threshold electrical signal can have an enhancement effect on neural activity, including nerve conduction. The sub-threshold electrical signal, in some instances, can have an enhancement effect on neural activity when applied by itself without blocking. The sub-threshold electrical signal, in other instances, can have an enhancement effect on neural activity when applied in conduction with a blocking electrical signal. The sub-threshold electrical signal, in still other instances, can have an enhancement effect on neural activity when applied after application of a blocking signal.

### IV. Examples

The following example describes procedure and results showing accelerated recovery of direct current (DC) blocking using repolarization. The following example is for the purpose of illustration only is not intended to limit the scope of the appended claims.

### Methods

### SINE Electrode Setup

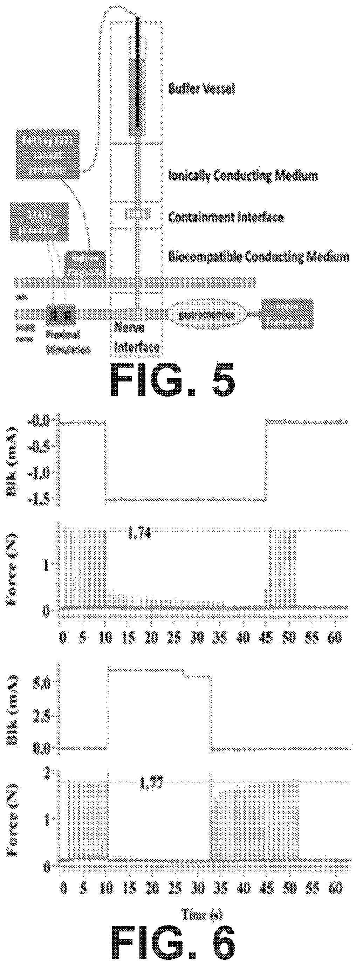

An acute experiment was conducted on one adult male rat 491 g (Sprague-Dawley), under institutional approval. The animal was anaesthetized with inhaled Isoflurane to effect. A SINE electrode was used for conduction block. To improve charge capacity, the vessel was filled with a high surface area carbon/saline "slurry". The metal electrode and the nerve cuff interface were physically separated with a column of electrolyte. Any reactions that occur at the metal electrode were contained in the electrolyte. To improve the capacity of the SINE electrode, high surface area carbon (YP-50, Kuraray, Canoga Park, CA, USA) was added to the saline to form a stiff paste. The carbon paste that was used to fill this buffer consisted of 3 g of carbon to 7 g of electrolyte (0.9 wt % saline). In this embodiment, a corrosion resistant graphite rod was used as the electrode contact. A syringe filter separated the carbon slurry from the biocompatible conducting medium, preventing the carbon from leaching down to the nerve. A silicone cuff was fabricated in house using silicone tubing to interface with the nerve. The cuff helps to hold the electrode in place close to the nerve. A current-controlled constant current generator (Keithley, Inc.) was used to provide both the depolarization and the repolarization cycles. A proximal stimulation electrode was placed proximally on the sciatic nerve to elicit muscle contractions from the gastrocnemius. Muscle contractions were measured by attaching the Achilles tendon to a force transducer. Proximal stimulation was provided by a Grass S88 Grass Technologies, West Warwick, RI, USA) stimulator through a voltage to current isolator at levels that produced a maximum muscle twitch (1Hz, 20µS, and 0.4-1mA). The blocking electrode was applied 10 mm distally through a separate incision and the return was a hypodermic needle placed subcutaneously on top of the biceps femoris muscle. An example preparation of the SINE electrode is shown in FIG. 5.

### in vivo Testing

Block threshold was defined as the lowest value at which complete block occurred within 30 s of application. The block thresholds using both a cathodic (negative) current and anodic (positive) current were determined.

Once the block thresholds were determined, the recovery time for complete block using the cathodic current was measured. The current was applied at the cathodic block threshold for 10 minutes. After 10 minutes, the current was turned off and the time for the force to completely recover was determined.

After the recovery time for complete block was determined, the accelerated recovery time was tested using various time periods of repolarization. First the current output was set to the block threshold for the cathodic current for 10 minutes of block. The output was then transitioned to 25% of the anodic block current for one of three time periods (90, 120, and 200 s). After this time period the block was turned off. The recovery time for the force was then recorded.

### Results

### Block Threshold

The negative polarity block threshold was determined to be -1.5 mA and the positive block threshold was determined to be +6.0 mA. FIG. 6 shows the complete block of force for values of -1.5 mA (top) and +6.0 mA (bottom). For the experiment, 25% of positive block threshold (1.5 mA) was used to test recovery acceleration.

### Test with No Recovery Acceleration

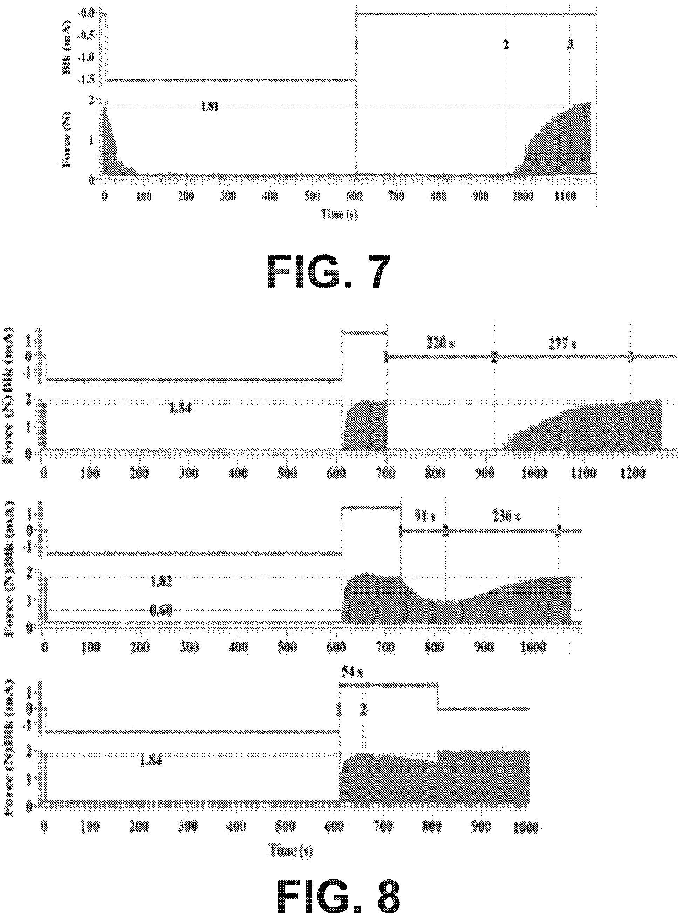

As shown in FIG. 7, complete block was achieved 60 s after application of a -1.5 mA DC block. After 10 minutes, recovery was monitored. Recovery of force twitches was achieved 357 s after the block was removed and complete force recovery occurred after an additional 152 s for a total recovery time of 509 s.

### Test with Recovery Acceleration

In FIG. 8, recovery acceleration is shown for repolarization periods of 90 s (top of FIG. 8), 120 s (middle of FIG. 8), and 200 s (bottom of FIG. 8). For the 90 s trial, force recovery occurred immediately, but then was reduced to 0 N when the DC was removed (0 mA). Initial force twitches can be seen DC was 220 s after the DC is removed with an additional 277 s for full force recovery. To determine the recovery times from the end of the 10 minutes of total block, the amount of time of repolarization (90 s) needs to be added for a twitch recovery for a twitch recovery time of 310 s and a full force recovery of 587 s.

For the 120 s trial, the force recovery is maintained after the DC is removed, but decays, and then eventually recovers. The twitch recovery after the removal of DC was 91 s with an additional 230 s for full force recovery. To determine the recovery times from the end of the 10 minutes' total block period, the amount of time of repolarization (120 s) needs to be added for a twitch recovery of 211 s and a full force recovery of 441 s.

For the 200 s trial, the repolarization phase actually starts to cause a conduction block (force reduction) after 54 s, and then complete recovery happens immediately when the DC is removed. In this example, sustained full force recovery occurs immediately after the repolarization interval for a full force recovery time of 200 s.

## Claims

1. A system (10, 20) comprising:
a current generator (14) configured to:
generate a DC of a polarity with an amplitude that is above a block threshold of a nerve for the polarity for a time, wherein the time is two minutes or more; and
after the time, generate another DC with an opposite polarity and another amplitude for another time, wherein the other amplitude is below the block threshold of the nerve for the opposite polarity; and
an electrode (12), coupled to the current generator (14), configured to deliver the DC to the nerve to block conduction in the nerve for the time and then deliver the other DC to the nerve for the other time so the nerve enters a repolarization cycle to accelerate a recovery time for the nerve.

2. The system (10, 20) of claim 1, wherein conduction in the nerve is fully restored after the other time.

3. The system (10, 20) of claim 1 or 2, wherein the subthreshold amplitude is less than 85 % of the threshold.

4. The system (10, 20) of claim 3, wherein the subthreshold amplitude is less than 50 % of the threshold.

5. The system (10, 20) of claim 4, wherein the subthreshold amplitude is less than 30 % of the threshold.

6. The system (10, 20) of any of the preceding claims, further comprising a controller (22) coupled to the current generator (14) configured to program the current generator (14) to generate the DC for the time and to generate that other DC for the second time.

7. The system (10, 20) of claim 6, wherein the controller (22) is implantable and/or external.

8. The system (10, 20) of any one of the preceding claims for use in a method to accelerate a recovery time for a nerve, the method comprising:
blocking conduction in the nerve by applying the DC to the nerve for the time; and
switching from the DC to the other DC with the opposite polarity to enter the repolarization cycle.

9. The system (10, 20) for use in the method of claim 8, wherein the nerve comprises at least two fiber types and wherein one of the at least two fiber types has a faster speed of recovery than another of the at least two fiber types.

## Patentansprüche

1. System (10, 20), Folgendes umfassend:
einen Stromgenerator (14), der zu Folgendem konfiguriert ist:
Erzeugen eines Gleichstroms einer Polarität mit einer Amplitude, die oberhalb eines Blockadenschwellenwerts eines Nervs für die Polarität für eine Zeitdauer liegt, wobei die Zeitdauer zwei Minuten oder mehr beträgt; und
nach der Zeitdauer, Erzeugen eines anderen Gleichstroms mit einer entgegengesetzten Polarität und einer anderen Amplitude für eine andere Zeitdauer, wobei die andere Amplitude unterhalb des Blockadenschwellenwerts des Nervs für die entgegengesetzte Polarität liegt, und
eine Elektrode (12), die an den Stromgenerator (14) gekoppelt ist, die dafür konfiguriert ist, den Gleichstrom an den Nerv zu liefern, um die Leitung in dem Nerv für die Zeitdauer zu blockieren und dann den anderen Gleichstrom an den Nerv für die andere Zeitdauer zu liefern, sodass der Nerv in einen Repolarisationszyklus eintritt, um eine Erholungsdauer für den Nerv zu beschleunigen.

2. System (10, 20) nach Anspruch 1, wobei die Leitung in dem Nerv nach der anderen Zeitdauer vollständig wiederhergestellt ist.

3. System (10, 20) nach Anspruch 1 oder 2, wobei die Amplitude unterhalb des Schwellenwerts weniger als 85 % des Schwellenwerts beträgt.

4. System (10, 20) nach Anspruch 3, wobei die Amplitude unterhalb des Schwellenwerts weniger als 50 % des Schwellenwerts beträgt.

5. System (10, 20) nach Anspruch 4, wobei die Amplitude unterhalb des Schwellenwerts weniger als 30 % des Schwellenwerts beträgt.

6. System (10, 20) nach einem der vorhergehenden Ansprüche, ferner umfassend eine Steuerung (22), die an den Stromgenerator (14) gekoppelt ist, die dafür konfiguriert ist, den Stromgenerator (14) zu programmieren, um den Gleichstrom für die Zeitdauer zu erzeugen und diesen anderen Gleichstrom für die zweite Zeitdauer zu erzeugen.

7. System (10, 20) nach Anspruch 6, wobei die Steuerung (22) implantierbar und/oder extern ist.

8. System (10, 20) nach einem der vorhergehenden Ansprüche zur Verwendung in einem Verfahren zum Beschleunigen einer Erholungsdauer für einen Nerv, wobei das Verfahren Folgendes umfasst:
Blockieren der Leitung in dem Nerv durch Aufbringen des Gleichstroms auf den Nerv für die Zeitdauer, und
Umschalten von dem Gleichstrom auf den anderen Gleichstrom mit der entgegengesetzten Polarität, um in den Repolarisationszyklus einzutreten.

9. System (10, 20) zur Verwendung in dem Verfahren nach Anspruch 8, wobei der Nerv mindestens zwei Fasertypen umfasst und wobei einer der mindestens zwei Fasertypen eine schnellere Erholungsgeschwindigkeit aufweist als ein anderer der mindestens zwei Fasertypen.

## Revendications

1. Système (10, 20) comprenant :
un générateur de courant (14) configuré pour :
générer un courant continu d'une polarité avec une amplitude qui est supérieure à un seuil de blocage d'un nerf pour la polarité pendant une durée, dans lequel la durée est de deux minutes ou plus ; et
après la durée, générer un autre courant continu avec une polarité opposée et une autre amplitude pendant une autre durée, dans lequel l'autre amplitude est inférieure au seuil de blocage du nerf pour la polarité opposée ; et
une électrode (12), couplée au générateur de courant (14), configurée pour délivrer le courant continu au nerf pour bloquer la conduction dans le nerf pendant la durée et ensuite délivrer l'autre courant continu au nerf pendant l'autre durée afin que le nerf entre dans un cycle de repolarisation pour accélérer un temps de récupération du nerf.

2. Système (10, 20) selon la revendication 1, dans lequel la conduction dans le nerf est complètement rétablie après l'autre durée.

3. Système (10, 20) selon les revendications 1 ou 2, dans lequel l'amplitude sous le seuil est inférieure à 85 % du seuil.

4. Système (10, 20) selon la revendication 3, dans lequel l'amplitude sous le seuil est inférieure à 50 % du seuil.

5. Système (10, 20) selon la revendication 4, dans lequel l'amplitude sous le seuil est inférieure à 30 % du seuil.

6. Système (10, 20) selon l'une quelconque des revendications précédentes, comprenant en outre un dispositif de commande (22), couplé au générateur de courant (14), configuré pour programmer le générateur de courant (14) pour générer le courant continu pendant la durée et générer cet autre courant continu pendant la deuxième durée.

7. Système (10, 20) selon la revendication 6, dans lequel le dispositif de commande (22) est implantable et/ou externe.

8. Système (10, 20) selon l'une quelconque des revendications précédentes pour utilisation dans un procédé d'accélération d'un temps de récupération d'un nerf, le procédé comprenant les étapes consistant à :
bloquer la conduction dans le nerf en appliquant le courant continu au nerf pendant la durée ; et
passer du courant continu à l'autre courant continu de polarité opposée pour entrer dans le cycle de repolarisation.

9. Système (10, 20) pour utilisation dans le procédé selon la revendication 8, dans lequel le nerf comprend au moins deux types de fibres et dans lequel l'un des au moins deux types de fibres présente une vitesse de récupération plus rapide qu'un autre des au moins deux types de fibres.
